# EUROPEAN PATENT SPECIFICATION

(11) **EP 1 571 203 B1**
(45) Date of publication and mention of the grant of the patent: **24.07.2013**
(21) Application number: 05100038.8
(22) Date of filing: 05.01.2005
(51) Int. Cl.: A61K 31/70

(54) **Anti-inflammatory active principles in Euganean thermal mud**
Prinzipien der Entzündungshemmung im euganeischer Thermalschlamm
Principes anti-inflammatoires actifs dans la boue thermale euganéenne

(30) Priority: 09.01.2004 IT MI20040011
(43) Date of publication of application: 07.09.2005
(73) Proprietor: CENTRO STUDI TERMALI VENETO PIETRO D'ABANO DI ABANO TERME MONTEGROTTO TERME, 35031 Abano Terme (IT)
(72) Inventor: Lalli, Alberto, 35031 Abano Terme (IT); Andreoli, Carlo, 35020 Albignasego (IT); Ceschi Berrini, Cristina, 35127 Padova (IT); De Appolonia, Francesca, 33080 Prata di Pordenone (IT); Marcolongo, Gabriele, 35020 Due Carrare (IT)
(74) Representative: Gervasi, Gemma

(56) References cited:
- JP-A- 2002 338 474
- BERRINI CRISTINA CESCHI ET AL: "Morphological and molecular characterization of a thermophilic cyanobacterium (Oscillatoriales) from the Euganean Thermal Springs (Padua, Italy)" ARCHIV FUER HYDROBIOLOGIE SUPPLEMENT, vol. 153, no. August, August 2004 (2004-08), pages 73-85, XP008050516 -& DATABASE EMBL [Online] 28 February 2003 (2003-02-28), "Phormidium sp. ETS-05 partial 16S rRNA gene, isolate ETS-05" XP002339467 retrieved from EBI accession no. EM_PRO:PSP548503 Database accession no. AJ548503
- CANTO DE LOURA I ET AL: "THE EFFECTS OF NITROGEN DEFICIENCY ON PIGMENTS AND LIPIDS OF CYANOBACTERIA" PLANT PHYSIOLOGY (ROCKVILLE), vol. 83, no. 4, 1987, pages 838-843, XP002339465 ISSN: 0032-0889
- SHIRAHASHI H ET AL: "ISOLATION AND IDENTIFICATION OF ANTI-TUMOR-PROMOTING PRINCIPLES FROM THE FRESH-WATER CYANOBACTERIUM PHORMIDIUM TENNUE" CHEMICAL AND PHARMACEUTICAL BULLETIN, PHARMACEUTICAL SOCIETY OF JAPAN, TOKYO, JP, vol. 41, no. 9, 1993, pages 1664-1666, XP002928276 ISSN: 0009-2363
- TOLOMIO CLAUDIO ET AL: "Diatoms in the thermal mud of Abano Terme, Italy (Maturation period)" ARCHIV FUER HYDROBIOLOGIE SUPPLEMENT, vol. 143, June 2002 (2002-06), pages 11-27, XP008050533
- TOLOMIO CLAUDIO ET AL: "Thermophilic microalgae growth on different substrates and at different temperatures in experimental tanks in Abano Terme (Italy)" ARCHIV FUER HYDROBIOLOGIE SUPPLEMENT, vol. 150, no. February, February 2004 (2004-02), pages 145-157, XP008050515
- TOLOMIO CLAUDIO ET AL: "Colonization by diatoms and antirheumatic activity of thermal mud" CELL BIOCHEMISTRY AND FUNCTION, vol. 17, no. 1, March 1999 (1999-03), pages 29-33, XP002347147 ISSN: 0263-6484
- GALZIGNA L ET AL: "Thermal mud-pack as an anti-inflammatory treatment" BIOMEDICINE AND PHARMACOTHERAPY, vol. 52, no. 9, October 1998 (1998-10), pages 408-409, XP002347148 ISSN: 0753-3322
- GIROUD C ET AL: "LIPIDS OF CHLAMYDOMONAS-REINHARDTII ANALYSIS OF MOLECULAR SPECIES AND INTRACELLULAR SITES OF BIOSYNTHESIS" PLANT AND CELL PHYSIOLOGY, vol. 29, no. 4, 1988, pages 587-596, XP008050625 ISSN: 0032-0781
- WANG ZE-NENG ET AL: "Effects of Na2S2O3 and glucose on the compositions of glycerolipids and their fatty acids in Synechocystis sp. PCC 6803 cells." ACTA BOTANICA SINICA, vol. 45, no. 11, November 2003 (2003-11), pages 1339-1345, XP002347149 ISSN: 0577-7496
- GUSTAFSON K R ET AL: "AIDS-ANTIVIRAL SULFOLIPIDS FROM CYANOBACTERIA (BLUE-GREEN ALGAE)" JOURNAL OF THE NATIONAL CANCER INSTITUTE, US DEPT. OF HEALTH, EDICATIONAND WELFARE, PUBLIC HEALTH, US, vol. 81, no. 16, 16 August 1989 (1989-08-16), pages 1254-1258, XP001055127 ISSN: 0027-8874
- MURAKAMI N ET AL: "Studies on glycolipids. III. Glyceroglycolipids from an axenically cultured cyanobacterium, Phormidium tenue" CHEMICAL AND PHARMACEUTICAL BULLETIN, PHARMACEUTICAL SOCIETY OF JAPAN, TOKYO, JP, vol. 39, no. 9, September 1991 (1991-09), pages 2277-2281, XP002100388 ISSN: 0009-2363

## Description

### Field of the invention

Thermal mud has been used since ancient times for the treatment of inflammatory pathologies and in particular osteoarticular diseases.

The thermal complexes of Abano Terme, Montegrotto Terme and Battaglia Terme are particularly renowned for the therapeutic properties of their thermal mud and are found at the foot of the Euganean hills near Padua. Mud accumulates at the bottom of two thermal lakes, (Arqua' and Lispida), from where it is drawn and poured Into tanks where it undergoes a so called "maturation" process, during which it is incubated for 2 months in the presence of a continuous flow of spring water at a temperature of 85-87°C. During this process the mud is abundantly colonised by cyanobacteria and microalgae which impart valuable chemo-physical and therapeutic properties.

Shirahashi H. et al (Chem Pharm Bull (Tokyo) 1993 Sep;41 (9):1664-6) identified three classes of glycolipids extracted from cyanobacterium P. tenue. These glycolipids, namely: monogalactosyl diacylglycerol (MGDG), digalactosyl diacylglycerol (DGDG), and sulfoquinovosyl diacylglycerol (SQDG) were found to have an anti-tumor-promoting effect. Tolomio C. et al. (Cell biochemistry and function, vol. 17, no. 1, March 1999, pages 29-33) identified diatoms among other thermophilic microorganisms as the main agents for the colonization of thermal mud resulting in a 'maturation' which renders the mud suitable to be used for the treatment of osteoarthrosis. The diatom produces a sulfoglycolipid which has an anti-inflammatory action.

Gustafson K.R. et al (JNCI J Natl Cancer Inst (1989) 81 (16): 1254-1258) analysed extracts of cultured cyanobacteria (blue-green algae) and in particular the sulfonic acid-containing glycolipids, for inhibition of the cytopathic effects of the human immunodeficiency virus (HIV-1), which is implicated as a causative agent of AIDS.

Recently it has been demonstrated that the therapeutic effectiveness of mud bath treatment is not connected solely to the beneficial effects of heat and electrolytes but is due to the presence of substances possessing high anti-inflammatory activity, synthesised by the microorganisms which colonise the mud during the maturation process. The principal microorganisms present in Euganean thermal mud are cyanobacteria and microalgae. Among these one of the most abundant is a cyanobacterium belonging to the Oscillatoriales order.

### Summary of the invention

The current inventors have studied one of the most abundant cyanobacteria present in Euganean thermal mud and have surprisingly found that said microorganism, which belongs to the *Phormidium* genus and has been named ETS-O5, is different from known cyanobacteria present in other thermal mud.

The inventors have also found that said cyanobacterium produces glycolipid substances possessing an anti-inflammatory activity greater than or equivalent to -that of traditional anti-inflammatories but chararcterised by an absence of toxic effects.

Therefore, the present invention relates to cyanobacterium ETS-05 glycolipids with anti-inflammatory activity and to medicaments comprising said glycolipids.

A first embodiment of the present invention regards Use of glycolipids chosen from the group consisting of
a) monogalactosyldiglycerides containing a mixture of saturated and unsaturated fatty acids of between 14 and 18 carbon atoms characterised in that said mixture contains at least 45% of a saturated C16 acid and at least 25% of a tetraunsaturated C18 acid,
b) digalactosyldiglyceride containing a mixture of saturated and unsaturated fatty acids of between 14 and 18 carbon atoms, characterised in that said mixture contains at least 45% of a saturated C16 acid and at least 20 % of a tetraunsaturated C18 acid, and
c) a mixture of (a) and (b).
for the preparation of a medicament having anti-inflammatory activity, said medicament containing them as the active ingredients.

### Description of the figures

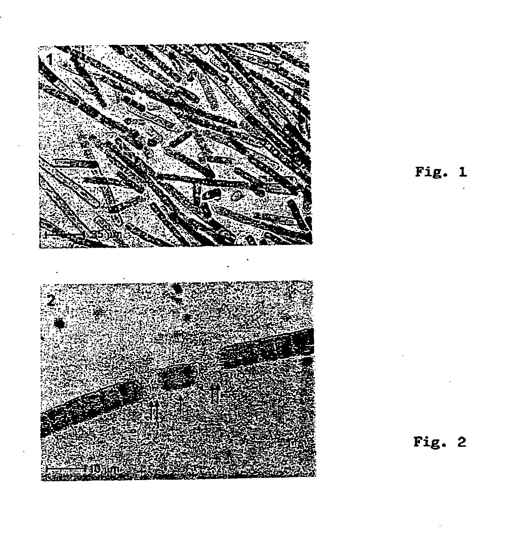

Figure 1 is an optical microscope image of cyanobacterium ETS-05 which shows the presence of solitary trichomes covered by a thin sheath, with no heterocysts.
Figure 2 is an optical microscope image of cyanobacterium ETS-05 which shows the formation of hormogonia (arrow) following fragmentation by necridia.

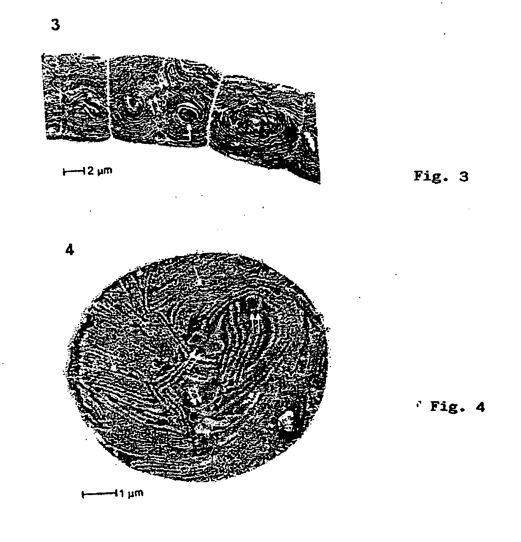

Figure 3 is an electron microscope image of cyanobacterium ETS-05 which shows a longitudinal section of a filament. Thylakoids occupy most of the cell volume. Cell division by fission is visible. The nucleoplasm (arrow) and the carboxysomes (double arrows) are visible.
Figure 4 is an electron microscope image of cyanobacterium ETS-05 which shows a transverse section of the cell. Thylakoids (arrow) are visible, organised in rows from the periphery to the centre of the cell. Some carboxysomes (double arrow) can be seen in the centre of the cell.

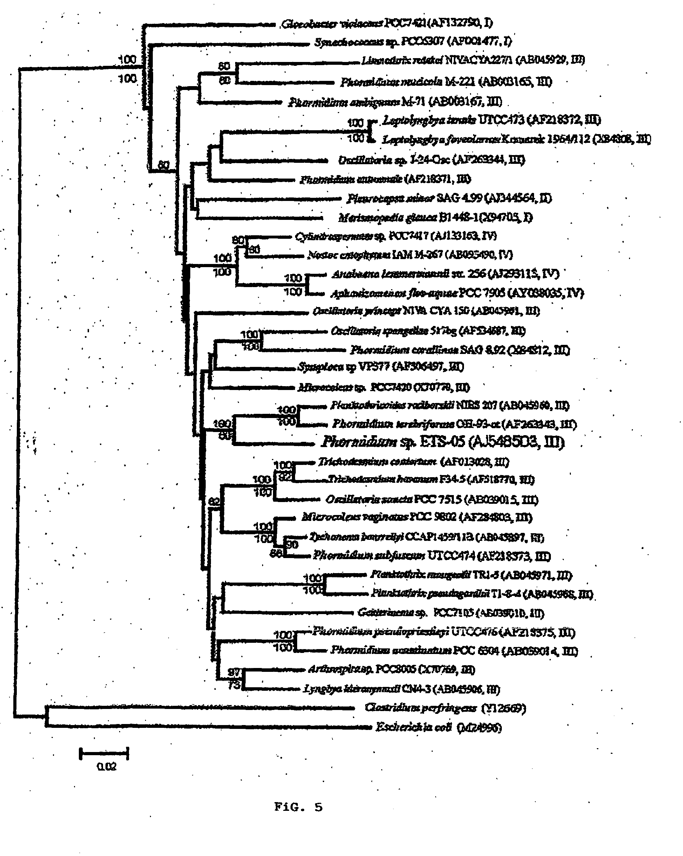

Figure 5 shows the phylogenetic tree based on the DNA sequence of the genes that code for 16S rRNA. The GenBank accession numbers and the order numbers of the cyanobacteria strains are indicated in inverted commas.

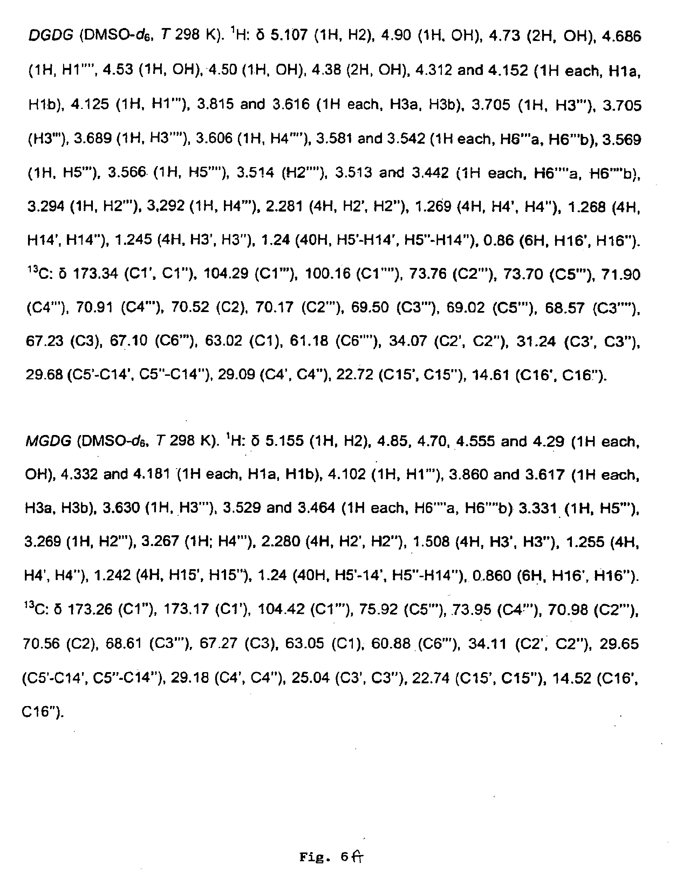

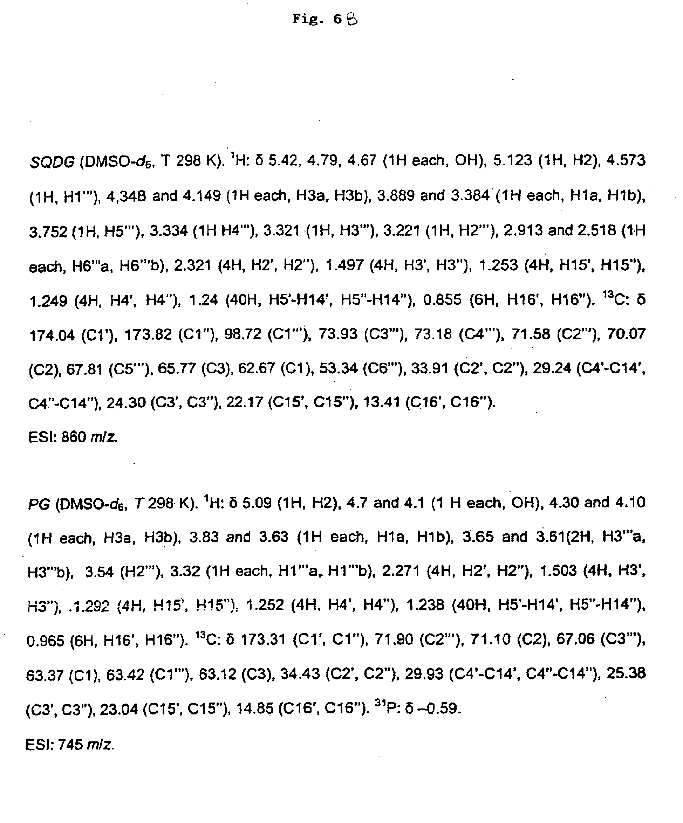

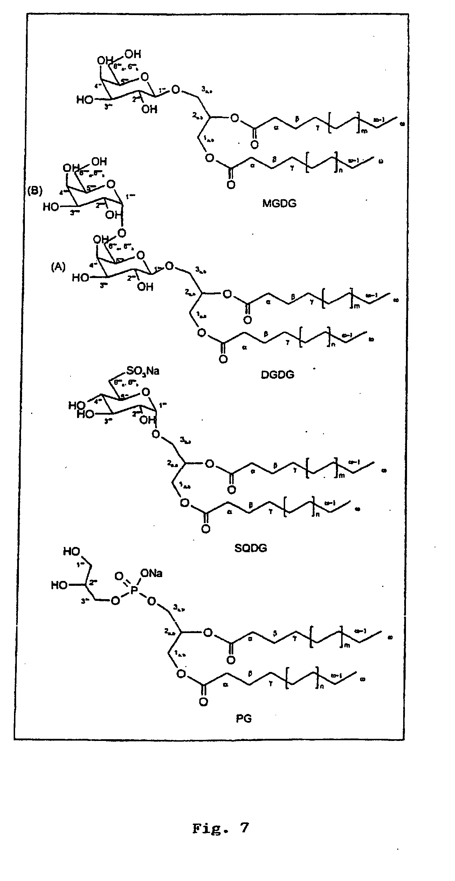

Figures 6A and 6B show the NMR spectrum of the glycolipids extracted from cyanobacterium ETS-05. The labelled positions correspond to those shown in Figure 7.
Figure 7 shows the structure of the glycolipids produced by cyanobacterium ETS-05.

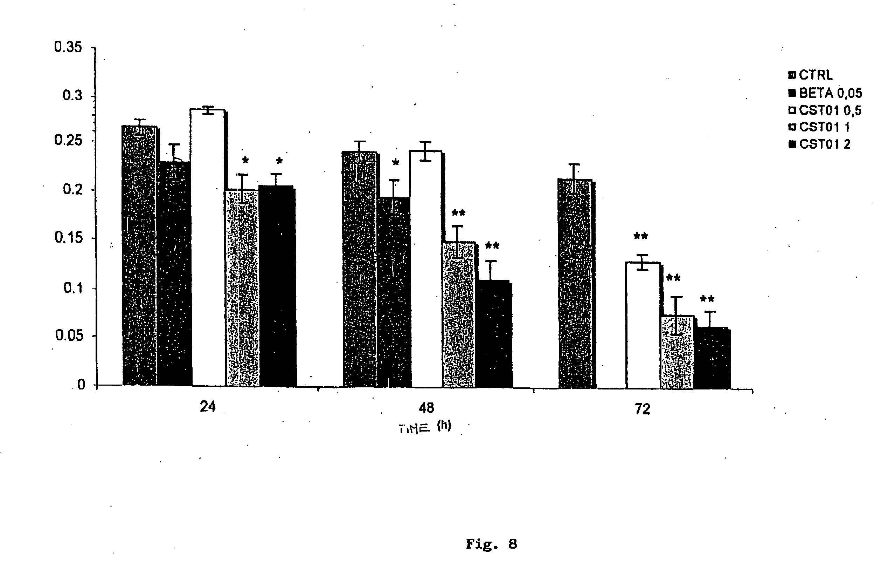

Figure 8 shows the anti-inflammatory effect of monogalactosyldiglycerides at a concentration of 0.5% (CTS01 0.5), 1% (CTS01 1) and 2% (CTS01 2), on the model of ear oedema induced by croton oil, compared to that of 0.05% betamethasone-17, 21-dipropionate (BETA 0.05). The results are gjven as the average change in mm of ear thickness compared to the base value at 24, 48 and 72 hours.

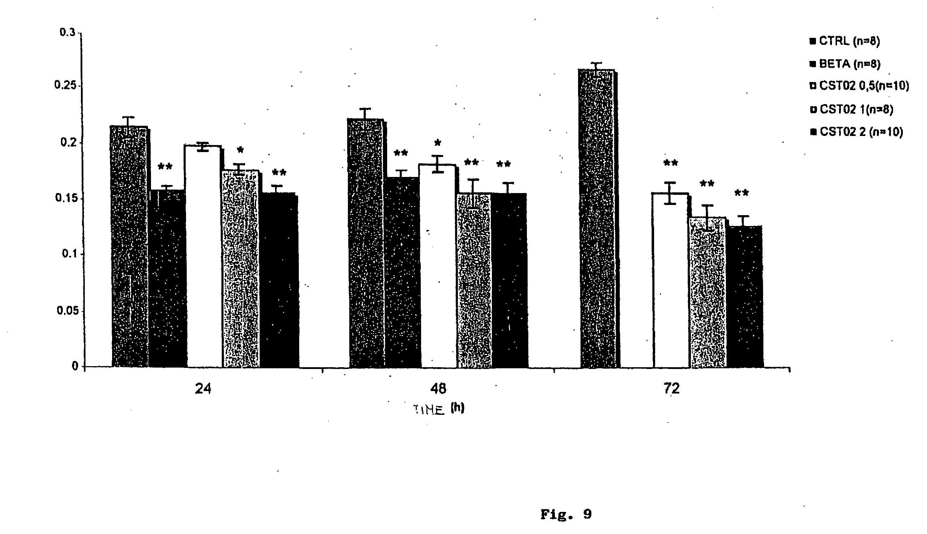

Figure 9 shows the anti-inflammatory effect of digalactosyldiglycerides at a concentration of 0.5% (CTS02 0.5), 1% (CTS02 1) and 2% (CTS02 2), on the model of ear oedema induced by croton oil, compared to that of 0.05% betamethasone-17, 21-dipropionate (BETA). The data are given as average change in mm of ear thickness compared to the base value at 24, 48 and 72 hours.

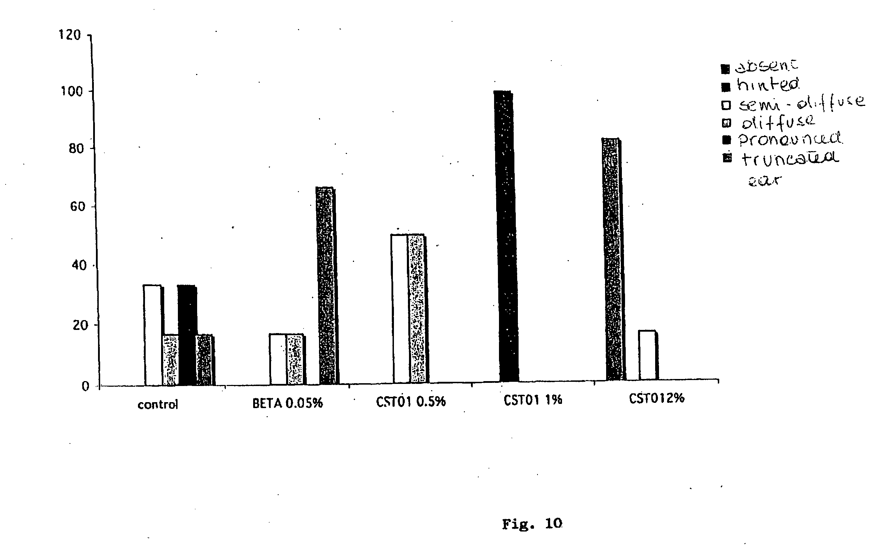

Figure 10 shows the preventive effect of monogalactosyldiglycerides at a concentration of 0.5% (CTS01 0.5%), 1% (CTS01 1 %) and 2% (CTS01 2%) on necrosis induced by croton oil compared to that of 0.05% betamethasone-17, 21-dipropionate (BETA 0.05%). The data are expressed as a percentage of the number of ears which display the phenomenon at each value assessed.

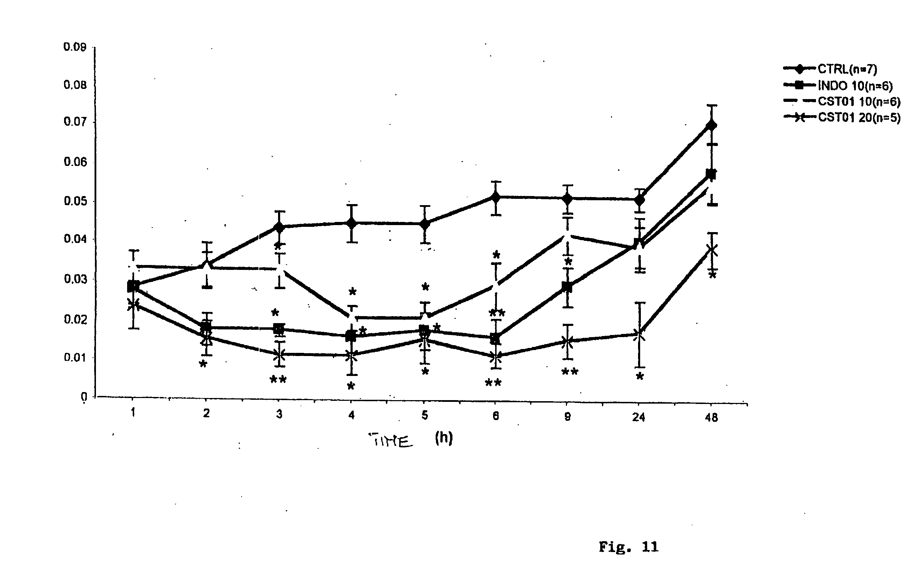

Figure 11 shows the anti-inflammatory effect of monogalactosyldiglycerides at doses of 10 mg/kg (CST01 10) and 20 mg/kg (CST01 20) on the model of acute paw oedema induced by carrageenan compared to that of indomethacin at a 10 mg/kg dose (INDO 10). The data are expressed as average change in paw volume (in ml) compared to the base value at different time intervals.

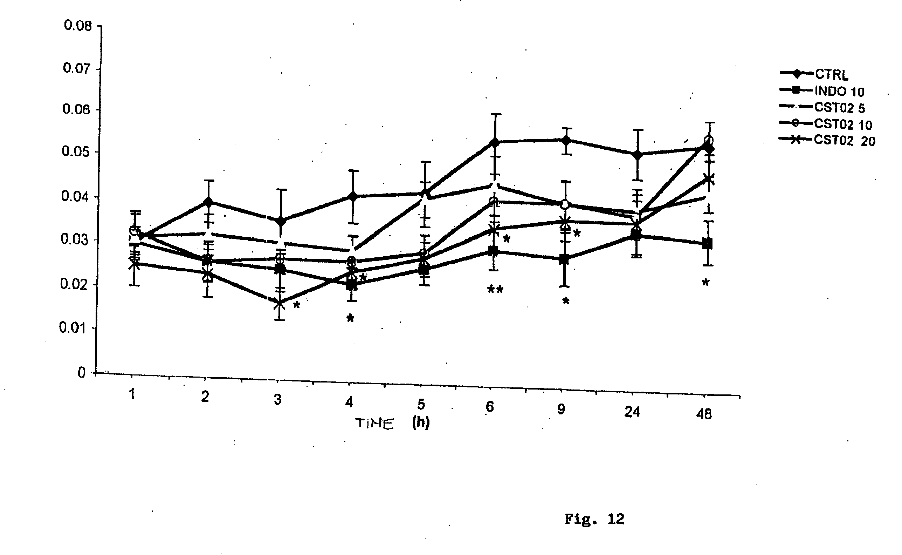

Figure 12 shows the anti-inflammatory effect of digalactosyldiglycerides at doses of 5 mg/kg (CST02 5), 10 mg/kg (CST02 10) and 20 mg/kg (CST02 20) on the model of acute paw oedema induced by carrageenan compared to that of indomethacin at a 10 mg/kg dose (INDO 10). The data are expressed as average change in paw volume (in ml) compared to the base value at different time intervals.

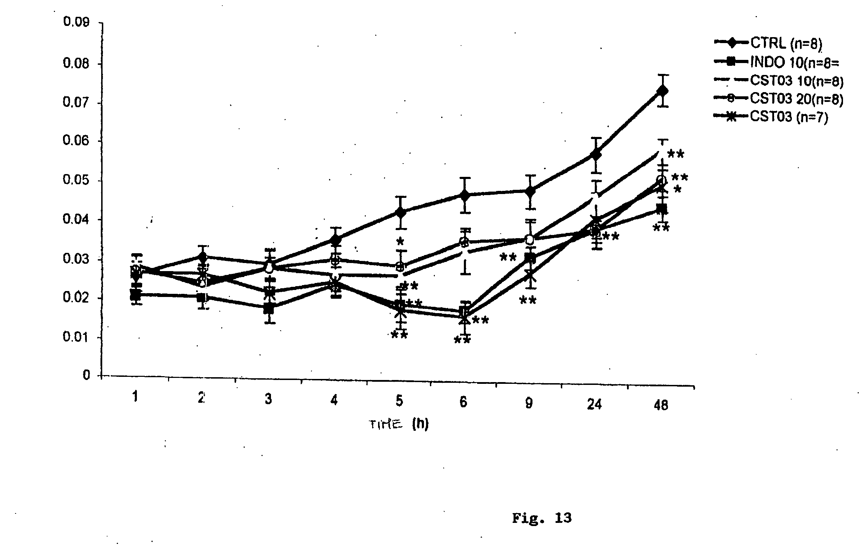

Figure 13 shows the anti-inflammatory effect of sulfoquinovosyldiglycerides at the dose of 10 mg/kg (CST03 10), 20 mg/kg (CST03 20) and 40 mg/kg (CST03 40) on the model of acute paw oedema induced by carrageenan compared to that of indomethacin at a 10 mg/kg dose (INDO 10). The data are expressed as average change in paw volume (in ml) compared to the base value at different time intervals.

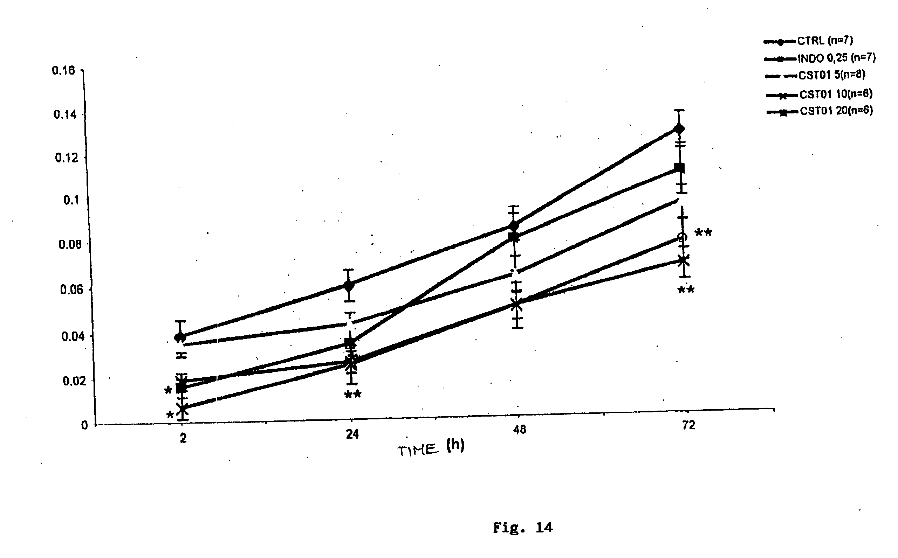

Figure 14 shows the anti-inflammatory effect of monogalactosyldiglycerides at a dose of 5 mg/kg (CST01 5), 10 mg/kg (CST01 10) and 20 mg/kg (CST01 20) on the model of chronic paw oedema induced by carrageenan compared to that of indomethacin at a 0.25 mg/kg dose (INDO 0.25). The data are expressed as average change in paw volume (in ml) compared to the base value at different time intervals.

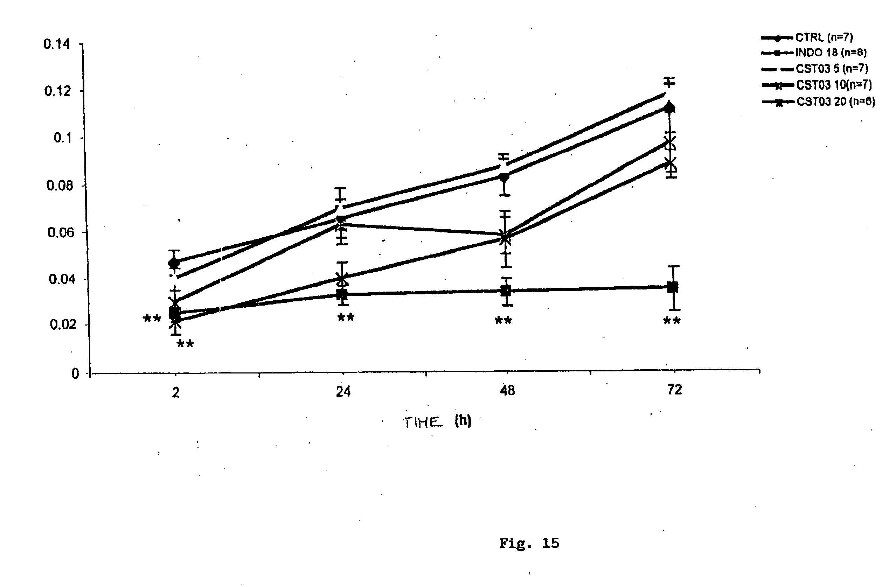

Figure 15 shows the anti-inflammatory effect of sulfoquinovosyldiglycerides at a dose of 5 mg/kg (GST03.5), 10 mg/kg (GST03 10) and 20 mg/kg (CST03 20) on the model of chronic paw oedema induced by carrageenan compared to that of indomethacin at a 18 mg/kg dose (INDO 18). The data are expressed as average change in paw volume (in ml) compared to the base value at different time intervals.

### Detailed description of the invention

Cyanobacterium ETS-05 is isolated from Euganean thermal mud, which is derived from the thermal lakes of Arqua and Lispida, by means of processes well known to the expert of the art, for example as illustrated in detail in example 1 of the experimental section.

As will be demonstrated in details in example 8 to follow, cyanobacterium ETS-05 can produce a mixture of glycolipids possessing anti-inflammatory activity greater than or equal to that of traditional anti-inflammatories, such as indomethacin and betamethasone, but unlike these they do not present any toxicity, even after repeated treatments.

A first embodiment of the present invention regards Use of glycolipids chosen from the group consisting of
d) monogalactosyldiglycerides containing a mixture of saturated and unsaturated fatty acids of between 14 and 18 carbon atoms characterised in that said mixture contains at least 45% of a saturated C16 acid and at least 25% of a tetraunsaturated C18 acid,
e) digalactosyldiglyceride containing a mixture of saturated and unsaturated fatty acids of between 14 and 18 carbon atoms, characterised in that said mixture contains at least 45% of a saturated C16 acid and at least 20 % of a tetraunsaturated C18 acid, and
f) a mixture of (a) and (b).
for the preparation of a medicament having anti-inflammatory activity, said medicament containing them as the active ingredients.

Preferably said glycolipids are used in a mixture.

Particularly preferred mixtures according to the present invention are the following:
a) mixtures consisting of monogalactosyidiglycerides whose composition of fatty acids is characterised by a saturated C16 acid content of at least 45% and a tetraunsaturated C18 acid content of at least 25%, preferably mixtures having the following composition of fatty acids:
   between 1.5% and 2.5% of a saturated C₁₄ acid;
   between 45% and 55% of a saturated C₁₆ acid;
   between 1.5% and 2.5% of a monounsaturated C₁₆ acid;
   between 3% and 4.5% of a monounsaturated C₁₈ acid;
   between 1.5% and 2.5 of a diunsaturated C₁₈ acid;
   between 8% and 9% of a triunsaturated C₁₈ acid;
   between 25% and 35% of a tetraunsaturated C₁₈ acid.
b) Mixtures consisting of digalactosyldiglycerides whose composition of fatty acids is characterised by a saturated C16 acid content of at least 45% and a tetraunsaturated C18 acid content of at least 20%, preferably mixtures having the following fatty acid composition:
   between 0 % and 1 % of a saturated C₁₄ acid;
   between 45% and 55% of a saturated C₁₆ acid;
   between 2.5% and 3.5% of a monounsaturated C₁₆ acid;
   between 0% and 1 % of a diunsaturated C₁₆ acid;
   between 0% and 1% of a triunsaturated C₁₆ acid;
   between 1 % and 2% of a saturated C₁₈ acid;
   between 1.5% and 2.5% of a monounsaturated C₁₈ acid;
   between 3.5% and 4.5% of a diunsaturated C₁₈ acid;
   between 10% and 12% of a triunsaturated C₁₈ acid;
   between 20 and 30% of a tetraunsaturated C₁₈ acid
d) Mixtures of the aforesaid mixtures a) and b).

The aforesaid glycolipids and the aforesaid mixtures can be obtained from cyanobacterium ETS-05 using a fermentation and extraction process well known to the expert in the art.

A further aspect of the present invention is the use of the aforesaid glycolipids and aforesaid mixtures for the preparation of a medicament for the therapy of inflammatory pathologies, preferably of osteoarticular pathologies. Preferably said medicament is used for locoregional applications, even more preferably topical.

The present invention relates in addition to a medicament comprising as active principle the aforesaid glycolipids or the aforesaid mixtures in combination with suitable excipients and/or diluents. Preferably said medicament has a formulation suitable for locoregional use, even more preferably for topical use.

Particularly preferred is a medicament for topical use which comprises directly the Euganean thermal mud mixed with suitable excipients and diluents.

The current inventors have found in addition that the growth of the cyanobacterium ETS is optimal at a temperature between 30 and 35°C, preferably between 31 and 33°C, and preferably in the presence of continuous light (10µmol m⁻² S⁻¹).

The aforesaid maturation process leads to the attainment of a Euganean thermal mud enriched with the cyanobacterium ETS-05 and therefore possessing a greater anti-inflammatory activity than the same mud matured under conditions known in the art.

The present invention will be illustrated in greater detail by the examples to follow:

### EXAMPLE 1

### Isolation and culture of cyanobacterium ETS-05

A sample of thermal mud was collected on the surface of the mud maturation tank in one of the establishments in the Euganean thermal area.

The sample was brought into the laboratory and, after observation with an optical microscope, was inoculated into culture media with different compositions of nutrients specific for the growth of cyanobacteria. After some days cyanobacterium ETS-05 was isolated from the culture media by picking single filaments, under a microscope, using the micropipette technique. The filaments were inoculated in test tubes containing the culture medium BG11 which was found to be the most suited to Phormidium cell growth. After some days the monospecificity of the isolated samples was verified.

Single filaments of ETS-05 were isolated with a micropipette and grown in Erlenmayer flasks, in artificially enriched media, under constant agitation and continuous light (10µmol m⁻² s⁻¹) at a temperature of 31-33°C for 24 hours.

The temperature of 31-33°C is optimal for cyanobacterium growth and was selected in a study undertaken in the range between 20° and 50°C.

### EXAMPLE 2

### Morphological characterisation of the isolated strain

The morphological characteristics of the cyanobacterium ETS-05 isolated in example 1 were assessed by optical and electron microscope examination.

### Materials and methods

A Leica Dialux 22 microscope equipped with a Wild MPS51 camera was used for optical microscope analysis.

For the analysis by electron microscope a Hitachi HS9 microscope operating at 75 kV was used.

For the analysis by electron microscope the cells were fixed in 3% glutaraldehyde in 0.1 M cacodylate buffer (pH 6.9) for three hours at approximately 18°C. After being washed in three changes of 0.1 M cacodylate buffer, the specimens were fixed in 1 % OsO₄ for 2 hours, dehydrated in a graded ethanol series, followed by propylene oxide. The specimens were then stained with uranyl acetate while undergoing dehydration in 75% ethanol. The specimens were then embedded in an Epon-Durcupan ACM mixture. Thin sections, cut with a Reichert Ultracut S, were stained with lead citrate and analysed.

### Results

As can be seen from Figure 1, using the optical microscope cyanobacterium ETS-05 shows solitary trichomes, with no heterocysts. When the trichomes are bent a slow wave-like movement of the terminal part of the trichome and an axial movement are visible. Figure 2 shows the typical cell division due to fragmentation by necridia; this leads to the development of hormogonia, these being usually short and characterised by a sheath that is open at its ends and that features rounded terminal cells. The fully grown and developed trichomes are isopolar with morphologically identical ends, but after fragmentation they acquire a different polarity, showing pointed and curved terminal cells at one end and rounded cells at the other end.

Cell division takes place by fission along a plane perpendicular to the longitudinal axis of the trichome.

This is evident also from the electron microscope images (Fig 3) in which the continuity of the thylakoids upstream of cell wall completion and their sudden interruption downstream thereof can be noted. The daughter cells divide and grow into their mother cell size before the next division. Several thylakoids can be noticed in each cell; they take up the greatest part of the cell volume and have several phycobilisomes. However, whereas in longitudinal section the thylakoids are arranged in several parallel rows concentric to the nucleoplasm (Fig 3), in cross section they appear irregularly rolled up in the centre and situated perpendicular to the cell wall toward the periphery (Fig 4). Some carboxysomes can be seen at the centre of the cells in both Figure 3 and 4. No gas vacuoles were observed.

Optical and electron microscope observation of cells taken from the natural environment of ETS-05 has demonstrated that these are identical to the cultured cyanobacterium in terms of both its morphometric aspects and ultrastructural characteristics.

The morphometric characteristics of the cyanobacterium ETS-05 cells in culture (A) and those taken from the natural environment (B) are summarised in the following table 1:

**Table 1**

| Morphotype | A | B |
|---|---|---|
| Thallus | solitary trichomes | solitary trichomes |
| Colour of trichomes | emerald green | emerald green |
| Sheath | thin | thin |
| Cell length | 4.6±0.6 µm | 5.1±0.5 µm |
| Cell width | 4.8±0.2 µm | 5.0±0.3 µm |
| Hormogonia formation | Fragmentation by necridia | Fragmentation by necrid ia |
| Motility | Oscillatory motion | Oscillatory motion |

The morphological and ultrastructural characteristics of cyanobacterium ETS-05 show that it is probably a species belonging to the *Phormidium* genus.

The arrangement of thylakoids corresponds precisely to that of several species of the Phormidium genus and of all the Phormidiaceae. Moreover, the strain ETS-05, as with the other Phormidium species, does not possess aerotopes which are instead present in Planktothrix, Planktothricoides and in Trichodesmium, is unable to fix atmospheric nitrogen unlike the species belonging to the genus Trichodesmium and Symploca and, in contrast to Porhyrosiphon and Pseudophormidium, does not show respectively a rigid sheath and false branching. Finally, its ultrastructural and cell dividing characteristics differ from those observed in Tychonema.

### EXAMPLE 3

### Phylogenetic analysis

The cultured cells from example 1 were harvested by centrifugation and the genomic DNA was extracted using the DNeasy Tissue kit from Qiagen. Amplification and sequencing of the DNA coding for ribosome 16S (16S rDNA) of cyanobacterium ETS-05 was undertaken using the primers given in table 2, which were developed by the inventors based on alignments of known cyanobacterial 16S rDNA sequences (*Phormidium ambiguum,* AB003167; *Stanieria cyanosphaera*, AF132931; *Anabaena cilindrica*, AF091150; *Nodularia sp.*, PCC9350; AY038034; *Trichodesmium erythraeum*, AF013030; *Oscillatoria sancta*, AF132933, *Synechococcus elongatus*, D83715), except for primers 16S-1 and 16S-2 which were designed by Kobayashi (A.N.: D83715).

| Primer | Sequence | Position | GenBank |
|---|---|---|---|
| 16S-1 | 5'-AGAGTTTGATCCTGGCTCAG-3' (SEQ ID No 2) | +1-+20 | D83715 |
| 16S-2 | 5'-ACGGCTACCTTGTTACGACTT-3' (SEQ ID No 3) | +1453-+1433 | D83715 |
| 16S-3 | 5'-CTACGGGAGGCAGCAGTG-3' (SEQ ID No 4) | +304-+321 | AJ548503 |
| 16S-4 | 5'-CACTGCTGCCTCCCGTAG-3' (SEQ ID No 5) | +321-+304 | AJ548503 |
| 16S-5 | 5'-ATACCCCAGTAGTCCTAG-3' (SEQ ID No 6) | +731-+748 | AJ548503 |
| 16S-6 | 5'-TAAACCACATACTCCACC-3' (SEQ ID No 7) | +898-+881 | AJ548503 |

In detail, the 16S rDNA gene was amplified as a single fragment from the DNA extract by the PCR technique using the primers 16S-1 and 16S-2.

A 100 µl reaction volume was used containing 10 mM TRIS-HCl (pH 8.3), 50 mM KCI, 1.5 mM MgCl₂, 0.2 mM of each dNTP, 0.25 µM of 3' and 5' primers and 2.5 U of Taq DNA polymerase (Celbio).

The amplification conditions were the following:
2 minutes at 95°C, followed by 40 cycles as follows:
   45 seconds at 95°C,
   30 seconds at 58°C. and
   45 seconds at 72°C
and by a 10 minutes cycle at 72°C.

The PCR products were separated on 1% agarose gel. The band obtained was excised from the gel and the DNA was extracted using the Jetsorb Gel Extraction Kit (Celbio). The PCR product was sequenced using the ABI PRISM Dye Terminator Cycle Sequencing Core Kit (Applied Biosystems) covering the entire length of the amplification product in both directions. Electrophoresis of the sequencing reactions was completed on an ABI PRISM model 377, version 2.1.1. automated sequencer

The sequence identified corresponds to SEQ ID No. 1.

The 16S rDNA sequence was aligned with the other sequences of cyanobacteria available in GenBank, using CLUSTALW software.

In particular, the sequence was aligned with 27 Oscillatoriales sequences 1100-1500 base pairs in length, with the sequences of some species belonging to the Chroococcales (I), Pleurocapsales (II), Nostrocales (IV) and Stigonematales (V) orders and with the sequence of Escherichia coli and Clostridium perfringens as outgroup.

The 16S rDNA sequence of the cyanobacterium ETS-05 is different from that of the other thermal Oscillatoriales.

Two different methods were used for phylogenetic tree construction: "neighbour joining" (NJ) and "maximum parsimony" (MP).

For the NJ method Kimura's 2-parameter mode! was used whereby all nucleotide positions containing gaps or missing data were deleted. To evaluate the robustness of branches in the phylogenetic tree the "Bootstrap (BT) Resampling method" of Felsenstein with 2000 replicates was used.

The phylogenetic tree obtained, shown in Fig. 5, demonstrates that the cyanobacterium ETS-05 is a species belonging to the *Phormidium* genus. In particular, the cyanobacterium ETS-05 is a "sister taxon" of the group formed from *Planktothricoides raciborskii* and *Phormidium terebiforme*, thermophylic species found in many hot springs; all these taxon belong in fact to the same phylogenetic group. Other thermophylic taxon, such as Oscillatoria sp. J-24-Osc form groups which are well separated both genetically and morphologically from that of *Phormidium* sp ETS-05 and *Phormidium terebriforme*.

### EXAMPLE 4

### Isolation and analysis of glycolipids produced from cyanobacterium ETS-05.

A culture of cyanobacterium ETS-05 was prepared as described in Example 1. The cultures were continuously monitored by weighing dry cells. The organic material was then harvested by sedimentation and filtration in the late exponential -early stationary phase.

The collected material was freeze-dried. Three different batches of lyophilised cells were produced to obtain a yield of between 300 and 350 mg of cells (dry weight) per litre of culture. 1 g of each batch of lyophilisate obtained was suspended in 25 ml of a chloroform/methanol/water mixture (in a 100:50:7 ratio). This mixture was treated for one minute with a turbine homogeniser (Ultraturrax T25, Janke and Kunkel IKA Labortechnik), then stirred with a magnetic stirrer for 30 minutes and filtered. The solid residue was re-extracted twice with 10 ml of the same solvent mixture, then discarded. The extracts were pooled, washed with 15 ml of water and then the solvent was evaporated with a rotavapor to achieve a dry product.

The residue was then reconstituted with 20 ml of methanol, 20 ml of n-hexane and 2 ml of water. The lower layer was re-extracted twice with 10 ml of n-hexane. The n-hexane extracts, containing neutral lipids and pigments were discarded whereas the methanol/water solution, containing the polar lipids, was subjected to evaporation of the solvent with a rotavapor to achieve a dry product. The residue (polar lipid fraction) was dissolved in 15 ml of a 2:1 chloroform/methanol mixture; this solution was then used for the subsequent quantitative analyses.

An equivalent lipid fraction obtained by the same procedure was fractionated by flash chromatography in a 250mm x 25 mm ID silica gel column. The elution was first carried out with 400 ml of a chloroform/methanol/water/acetic acid mixture in a 90:10:2.5:0.5 ratio and then with 1600 ml of a chloroform/methanol/water/acetic acid mixture in a 80:20:2:0.5 ratio. The eluted fractions were analysed by NMR. The fractions containing individual molecules of pure diacylglycerolipids were pooled, evaporated to dryness with a rotavapor and finally dried under high vacuum in the presence of P₂O₅, to be then submitted to structural analysis. Glycolipids were the main components found in the polar lipids. All the above steps were carried out in the dark, under cold nitrogen atmosphere.

### EXAMPLE 5

### NMR Analysis

The ¹H, ³¹P and ¹³C NMR spectra of the glycolipids obtained in example 4 as DMSO-d6 solution, were obtained by means of a Bruker Avance-600 spectrometer equipped with a TX-1 5 mm inverse probe operating at 600.18, 242.96 and 150.91 MHz respectively.

Assignment of proton resonances was performed by standard NMR correlations and by COSY, TOCSY; ROESY and NOESY experiments. The proton resonance of the OH group was found and attributed through bi-dimensional EXSY measurements, usually employed to detect solution dynamics in the slow motion region. The ¹³C resonances were observed and attributed through heterocorrelated COSY experiments (HMQC with bird sequence); quadrature along F1 was achieved by TPPI for hydrogen bonded carbon atoms and by HMBC for quaternary carbon atoms.

The results obtained are shown in Figures 6A and 6B, in which the positions indicated correspond to those of Figure 7.

The NMR spectra demonstrate that cyanobacterium ETS-05 produces four different diacylglycerides: monogalactosyldiglycerides (MGDG), digalactosyldiglycerides (DGDG), sulfoquinovosyldiglycerides (SQDG) and phosphatidyigiycerol (PG), shown in figure 7.

### EXAMPLE 6

### Quantitative analysis

0.150 ml of a polar lipid fraction solution were deposited on a silica gel TLC plate, 20x20 cm, 0.25 mm thick, together with standards of individual diacylglycerolipids. A mixture of chloroform/methanol/water/acetic acid (85:15:10:3) was used as eluent. The standard line was highlighted with iodine; the silica gel containing the individual lipids was scraped and extracted twice with 5 ml of a chloroform/methanol 2:1 mixture. The extracts were dried by evaporation. The glycolipids were measured against a galactose standard. The phosphatidylglycerol was measured against a phosphorus standard, after mineralisation with HClO₄.

The results obtained are summarised in table 3. Each reported value is the mean of three measurements.

| Compound | Batch 1 | | Batch 2 | | Batch 3 | |
|---|---|---|---|---|---|---|
| | Mol% | S.D. | Mol% S.D. | | Mol% | S.D. |
| MGDG | 50.1 | 7.07 | 48.61 | 2.45 | 49.39 | 5.61 |
| DGDG | 17.38 | 4.67 | 17.05 | 2.66 | 16.51 | 4.25 |
| SQDG | 21.90 | 1.72 | 23.07 | 6.23 | 23.60 | 1.52 |
| PG | 10.71 | 1.35 | 11.28 | 1.20 | 10.50 | 0.67 |

As can be seen from the table, MGDG comprises about 50% of glycerolipids, DGQG is slightly below 20%, SQDG is slightl over 20% and PG is around 10%.

### EXAMPLE 7

### Fatty acid composition analysis

The lipids were methylated with 14% boron trifluoride-methanol (w/v). Fatty acid methyl esters were identified by comparison with standards using gas chromatography in capillary columns.

The results obtained are given in the following table:

| fatty acids | MGDG Comp.% | DGDG Comp.% | SQDG Comp.% | PG Comp.% |
|---|---|---|---|---|
| 14:0 | 1.93 | 0.49 | 1.17 | 1.16 |
| **16:0** | **51.94** | **52.61** | **76.95** | **50.52** |
| 16:1 | 1.97 | 2.83 | 2.99 | 2.39 |
| 16:2 | 0.00 | 0.39 | 2.01 | 1.34 |
| 16:3 | 0.00 | 0.48 | 0.83 | 1.06 |
| 18:0 | 0.00 | 1.23 | 2.97 | 3.02 |
| 18:1 | 3.73 | 1.74 | 3.32 | 8.85 |
| 18:2 | 1.82 | 4.06 | 3.75 | 15.14 |
| 18:3 n-6 | 5.26 | 4.16 | 0.58 | 0.48 |
| 18:3 n-3 | 3.18 | 6.49 | 3.92 | 14.58 |
| **18:4 n-3** | **30.17** | **25.51** | **1.52** | **1.48** |

The main fatty acids contained in the four diacylglycerides are C16 and C18 acids with predominance of palmitic acid (16:0). MGDG and DGDG are characterised by a marked presence of polyunsaturated fatty acids, mainly C18:4.

### EXAMPLE 8

### Evaluation of anti-inflammatory activity

The compounds MGDG, DGDG and SQDG were obtained as described in example 4. The following solutions were then prepared:
142 mg of MGDG were dissolved in 5 ml of absolute ethanol (Carlo Erba) and brought to with a 0.5% Tween-80 solution (Merck) in physiological solution.
70 mg of SQDG were dissolved in 7 ml of absolute DMSO (Carlo Erba) with the help of an ultrasound device and divided into 10 aliquots of 700 µl. Each aliquot was then brought to a volume of 2 ml with physiological solution.
6 mg of DGDG were dissolved in 0.179 ml ethanol and brought to a volume of 3 ml with a 0.5% solution of Tween in physiological solution.

The anti-inflammatory activity of the compounds was evaluated using two experimental inflammation models in vivo:

### a) ear oedema induced by croton oil

This experimental model was used to test the anti-inflammatory activity for the compounds MGDG and DGDG only, since the compound SQDG is not soluble in ethanol. Groups of 3-5 animals were used. 3 µl of a solution of the substance to be tested were applied onto the ears of each animal, in concentrations between 0.5% and 3%, in a volatile carrier (ethanol).

In parallel, the control animals were treated with 3 µl of carrier, while the animals treated with the reference drug were treated with 3 µl of an 0.5% ethyl solution of betamethasone 17,21-diproprionate (SIGMA).

All the animals were treated twice a day, at intervals of 8 hours, for five days. Half an hour after the final treatment, 5 µl of a 20% croton oil solution in acetone were applied to the ears of each animal.

At different times following croton oil application the variation of the thickness of the ear compared to a base value shown immediately before the last treatment was evaluated using a digital micrometer (World Precision Instrument). In particular, in the preliminary stage of the experiment, ear thickness was measured 1, 2, 4, 6, 8, 24, 48 and 72 hours after the inflammatory stimulus; then having established that the oedema induced by croton oil attains its maximum peak after 24 and 48 hours it was considered sufficient to determine the variation in ear thickness at 24, 48 and 72 hours.

The statistical evaluation of the results was undertaken by determining the arithmetic mean for each group at each time interval and the relative standard error. The results were compared with the Dunnett test (P<0.05) and with the t-test (P<0.01).

Figures 8 and 9 show the average ear thickness variation observed with MGDG and DGDG respectively at 0.5%, 1 % and 2% concentrations.

As can be seen from figure 8, MGDG at the tested concentrations has demonstrated a significant capacity for reducing oedema, being greater than that observed with the reference compound betamethasone. In particular, 24 hours after the stimulus, at 1% and 2% concentrations, MGDG showed a reduction in oedema of 24% and 22% respectively, against a 15% reduction observed with betamethasone. After 48 hours a clear concentration dependent response was observed: the 1% and 2% preparations reduced oedema by 39% and 53% respectively, while the anti-inflammatory activity remained more or less unchanged in the case of betamethasone, for which the reduction remained at 19%. After 72 hours, even the lowest concentration of MGDG showed a statistically significant activity compared to the control, and reductions of 39%, 64% and 69% respectively were observed for the three concentrations tested.

MGDG was also tested at higher concentrations (3% and 5%) obtaining results comparable to those observed at the 2% concentration.

For the compound DGDG (figure 9) a dose dependent reduction, of 7%, 17% and 26% respectively, was observed 24 hours after the stimulus, therefore demonstrating, at the highest concentrations, an activity comparable to that of betamethasone. After 48 hours, a reduction of 29% was observed for the 1 % and 2% concentrations, this being greater than that observed with betamethasone (18%).

At 72 hours a clear concentration dependent response was observed, with reductions of 40%, 49% and 52% respectively.

In the case of DGDG it was not possible to test higher concentrations of the compound due to its low solubility.

As shown in figure 10, the *in vivo* experiment has highlighted the fact that ear tissue necrosis, representing the natural progression of the croton oil induced phenomenon, is considerably lower in the animals treated with the compounds to be tested compared to control animal and, in particular, animals treated with the reference drug. Said reduction is dose dependent and is greatest when the compound MGDG is used.

Therefore, the results obtained demonstrate that the compounds examined do not only show an anti-inflammatory effectiveness which is greater than betamethasone, but they are also able to prevent, in a dose dependent manner, tissue necrosis which is instead promoted by betamethasone.

### b) paw oedema induced by carrageenan.

Groups of 6-8 animals were used for the experiments.

Oedema in the right paw of each animal was induced by sub-cutaneous administration in the plantar region, of 50 µl of a 1% w/v solution of type λ carrageenan (FLUKA) in physiological solution.

In a preliminary step of the experiment, the animals were administered subcutaneously with doses from 5 to 80 mg/kg of the substance to be tested (MGDG, DGDG or SQDG), twice a day starting from three days before oedema induction and up to 72 hours afterwards (chronic model). The paw oedema was evaluated at different time intervals: 2, 24, 48 and 72 hours after the stimulus as variations compared to a base value, noted immediately before the carrageenan injection. The control animals were treated with the respective carrier, while the animals treated with the reference drug were administered with 0.25-18 mg/kg of indomethacin dissolved in physiological solution an hour prior to injecting with carrageenan and up to 72 hours afterwards, twice a day.

Subsequently, in the optimisation step of the model, the animals treated with the substance to be tested and with the indomethacin received a single sub-cutaneous administration half an hour before oedema induction (acute model). The control animals were simply injected with the carrier that was used. The variation in paw volume with respect to the base value was evaluated at the following time intervals: 1, 2, 3, 4, 5, 6, 9, 24 and 48 hours after the stimulus. Statistical evaluation of the results was undertaken by determining the arithmetic mean for each group at each time interval, and the relative standard error. The results were compared with the Dunnett test (P<0.05) and the t-test (P<0.01). Figures 11, 12 and 13 show the results obtained from the analysis of MGDG, DGDG and SQDG activities according to the aforedescribed acute model.

For the MGDG (figure 11) the 10, 20, 40 and 80 mg/kg doses were tested and an activity peak was observed at the 20 mg/kg dose. This dose showed a better activity than the indomethacin used in these experiments at a dose of 10 mg/kg. For the DGDG (figure 12) the 5, 10 and 20 mg/kg doses were tested and a clear dose dependent activity was observed. Contrary to DGDG, the anti-inflammatory activity, while being statistically significant between 3 and 9 hours after inflammatory stimulus, was however lower at the highest tested dose, compared to that of the reference drug.

For the SQDG (figure 13) 10, 20 and 40 mg/kg doses were tested. Only at the highest dose tested did the compound show anti-inflammatory activity comparable to that observed with indomethacin at the10 mg/kg dose.

Figures 14 and 15 show the results obtained from the analysis of MGDG and SQDG activity at the 5, 10 and 20 mg/kg doses according to the aforedescribed chronic model.

In the case of MGDG, 2 hours following stimulus, the percentages of paw volume reductions compared to those of control animals were, for the tested doses, 8%, 50% and 81 % respectively, whereby only the 20 mg/kg dose gave a reduction greater than the indomethacin 0.25/kg (reduction of 58%). At 24 hours even at the 10 mg/kg dose a greater reduction was observed than that obtained with the reference drug (30%, 57% and 58% respectively for the three MGDG dosages and 43% for indomethacin). At 48 and 72 hours all the tested doses were more active than indomethacin, with the greatest reduction for the 20 mg/kg dose (42% and 48% at 48 and 72 hours respectively).

In the case of SQDG, with the exception of the 20 mg/kg dose, which after 2 hours gave an oedema reduction of 53%, greater than that observed with the indomethacin at the 18 mg/kg dose (47%), an activity greater or comparable to the reference drug was never verified at 24, 48 and 72 hours, for any of the doses tested.

The indomethacin used as reference drug in the chronic experiments was found to be toxic at the 18 mg/kg dose, leading to loss of body weight and the deaths of half the animals treated before the experiment was concluded. This observation has rendered the use of a 10 mg/kg dosage necessary in subsequent experiments. The tested compounds, on the contrary, have not shown any sign of toxicity in any of the experiments conducted even though they were used in higher doses than indomethacin (up to 80 mg/kg).

## Claims

1. Use of glycolipids chosen from the group consisting of
a) monogalactosyldiglycerides containing a mixture of saturated and unsaturated fatty acids of between 14 and 18 carbon atoms **characterised in that** said mixture contains at least 45% of a saturated C16 acid and at least 25% of a tetraunsaturated C18 acid,
b) digalactosyldiglyceride containing a mixture of saturated and unsaturated fatty acids of between 14 and 18 carbon atoms, **characterised in that** said mixture contains at least 45% of a saturated C16 acid and at least 20 % of a tetraunsaturated C18 acid, and
c) a mixture of (a) and (b).
for the preparation of a medicament having anti-inflammatory activity , said medicament containing them as the active ingredients.

2. The use as claimed in claim1, **characterised in that** the mixtures (a) have the following fatty acid composition (a'):
between 1.5% and 2.5% of a saturated C₁₄ acid;
between 45% and 55% of a saturated C₁₆ acid;
between 1.5% and 2.5% of a monounsaturated C₁₆ acid;
between 3% and 4.5% of a monounsaturated C₁₈ acid;
between 1.5% and 2.5% of a diunsaturated C₁₈ acid;
between 8% and 9% of a triunsaturated C₁₈ acid;
between 25% and 35% of a tetraunsaturated C₁₈ acid.

3. The use as claimed in claim 1, **characterised in that** the mixtures (b) have the following fatty acid composition (b'):
between 0 % and 1% of a saturated C₁₄ acid;
between 45% and 55% of a saturated C₁₆ acid;
between 2.5% and 3.5% of a monounsaturated C₁₆ acid;
between 0% and 1% of a diunsaturated C₁₆ acid;
between 0% and 1% of a triunsaturated C₁₆ acid;
between 1% and 2% of a saturated C₁₈ acid;
between 1.5% and 2.5% of a monounsaturated C₁₈ acid;
between 3.5% and 4.5% of a diunsaturated C₁₈ acid;
between 10% and 12% of a triunsaturated C₁₈ acid;
between 20% and 30% of a tetraunsaturated C₁₈ acid.

4. The use according to anyone of claims 1-3, wherein said medicament having antiinflammatory activity is used in the treatment of inflammatory pathologies.

5. The use according to claim to claim 4, wherein said inflammatory pathologies are osteoarticular pathologies.

6. The use according to anyone of claims 1-5, wherein said medicament is suitable for topical administration.

## Patentansprüche

1. Verwendung von Glykolipiden, ausgewählt aus der Gruppe aufweisend:
a) Monogalaktosyl-Diglyceride, aufweisend ein Gemisch von gesättigten und ungesättigten Fettsäuren mit zwischen 14 und 18 Kohlenstoffatomen, **dadurch gekennzeichnet, dass** die Mischung zumindest 45% gesättigte C16 Säure und zumindest 25% einer vierfach-ungesättigten C18 Säure umfasst,
b) Digalaktosyl-Diglyceride aufweisend eine Mischung von gesättigten und ungesättigten Fettsäuren zwischen 14 und 18 Kohlenstoffatomen, **dadurch gekennzeichnet, dass** die Mischung zumindest 45% einer gesättigten C16 Säure und zumindest 20% einer vierfach-ungesättigten C18 Säure beinhaltet, und
c) eine Mischung von (a) und (b)
für die Aufbereitung eines Medikaments, aufweisend eine eine entzündungshemmende Eigenschaft, wobei das Medikament diese als aktive Zusatzstoffe aufweist.

2. Verwendung nach Anspruch 1, **dadurch gekennzeichnet, dass** die Mischung (a) die folgenden Fettsäurezusammensetzung (a') aufweist:
zwischen 1,5% und 2,5% einer gesättigten C14 Säure;
zwischen 45% und 55% einer gesättigten C16 Säure;
zwischen 1,5% und 2,5% einer einfach ungesättigten C16 Säure;
zwischen 3% und 4,5% einer einfach ungesättigten C18 Säure;
zwischen 1,5% und 2,5% einer doppelt ungesättigten C18 Säure;
zwischen 8% und 9% einer dreifach ungesättigten C18 Säure;
zwischen 25% und 35% einer vierfach ungesättigten C18 Säure.

3. Verwendung nach Anspruch 1, **dadurch gekennzeichnet, dass** die Mischung (b) die folgende Fettsäurezusammensetzung (b') aufweist:
zwischen 0% und 1% einer gesättigten C14 Säure;
zwischen 45% und 55% einer gesättigten C16 Säure;
zwischen 2,5% und 3,5% einer einfach gesättigten C16 Säure;
zwischen 0% und 1% einer zweifach ungesättigten C16 Säure;
zwischen 0% und 1% einer dreifach ungesättigten C16 Säure;
zwischen 1% und 2% einer gesättigten C18 Säure;
zwischen 1,5% und 2,5% einer einfach ungesättigten C18 Säure;
zwischen 3,5% und 4,5% einer zweifach ungesättigten C18 Säure;
zwischen 10% und 12% einer dreifach ungesättigten C18 Säure;
zwischen 20% und 30% einer vierfach ungesättigten C18 Säure.

4. Die Verwendung nach einem der Ansprüche 1 bis 3, bei der das Medikament mit einer entzündungshemmenden Eigenschaft in Behandlung von entzündlichen Pathologien verwendet wird.

5. Die Verwendung nach Anspruch 4, bei der die entzündlichen Pathologien osteoartikulare Pathologien sind.

6. Die Verwendung nach einem der Ansprüche 1 bis 5, bei der das Medikament geeignet ist für die topische Einnahme.

## Revendications

1. Utilisation de glycolipides sélectionnés dans le groupe consistant en
a) monogalactosyldiglycérides contenant un mélange d'acides gras saturés et insaturés d'entre 14 et 18 atomes de carbone, **caractérisée en ce que** ledit mélange contient au moins 45% d'acide C₁₆ saturé et au moins 25% d'acide C₁₈ tétra-insaturé,
b) digalactosyldiglycéride contenant un mélange d'acides gras saturés et insaturés d'entre 14 et 18 atomes de carbone, **caractérisée en ce que** ledit mélange contient au moins 45% d'acide C₁₆ saturé et au moins 20% d'acide C₁₈ tétra-insaturé, et
c) un mélange de (a) et (b)
pour la préparation d'un médicament ayant une activité anti-inflammatoire, ledit médicament les contenant en tant qu'ingrédients actifs.

2. Utilisation selon la revendication 1, **caractérisée en ce que** les mélanges (a) ont la composition d'acides gras suivante (a') :
entre 1,5% et 2,5% d'un acide C₁₄ saturé ;
entre 45% et 55% d'un acide C₁₆ saturé ;
entre 1,5% et 2,5% d'un acide C₁₆ mono-insaturé ;
entre 3% et 4,5% d'un acide C₁₈ mono-insaturé ;
entre 1,5% et 2,5% d'un acide C₁₈ di-insaturé ;
entre 8% et 9% d'un acide C₁₈ tri-insaturé ;
entre 25% et 35% d'un acide C₁₈ tétra-insaturé.

3. Utilisation selon la revendication 1, **caractérisée en ce que** les mélanges (b) ont la composition d'acides gras suivante (b') :
entre 0% et 1% d'un acide C₁₄ saturé ;
entre 45% et 55% d'un acide C₁₆ saturé ;
entre 2,5% et 3,5% d'un acide C₁₆ mono-insaturé ;
entre 0% et 1% d'un acide C₁₆ di-insaturé ;
entre 0% et 1% d'un acide C₁₆ tri-insaturé ;
entre 1% et 2% d'un acide C₁₈ saturé ;
entre 1,5% et 2,5% d'un acide C₁₈ mono-insaturé ;
entre 3,5% et 4,5% d'un acide C₁₈ di-insaturé ;
entre 10% et 12% d'un acide C₁₈ tri-insaturé ;
entre 20% et 30% d'un acide C₁₈ tétra-insaturé.

4. Utilisation selon l'une quelconque des revendications 1 à 3, dans laquelle ledit médicament ayant une activité anti-inflammatoire est utilisé dans le traitement de pathologies inflammatoires.

5. Utilisation selon la revendication 4, dans laquelle lesdites pathologies inflammatoires sont des pathologies ostéo-articulaires.

6. Utilisation selon l'une quelconque des revendications 1 à 5, dans laquelle ledit médicament convient pour l'administration topique.


### SEQUENCE LISTING

<110> Centro Studi Termali Veneto Pietro D'Abano di Abano Terme Montegrotto Terme
<120> ANTI-INFLAMMATORY ACTIVE PRINCIPLES IN EUGANEAN THERMAL MUD
<130> 4235PTEP
<150> IT MI2004A000011
   <151> 2003-01-09
<160> 1
<170> PatentIn version 3.1
<210> 1
   <211> 1414
   <212> DNA
   <213> Phormidium sp. ETS-05
<400> 1
   gatgaacgct ggcggtctgc ttaacacatg caagtcgaac ggaatccttc gggatttagt     60
   ggcggacggg tgagtaacgc gtgagaatct gccttcagga cggggacaac agttggaaac    120
   ggctgctaaa acccgatgtg ccgaaaggtg aaaaatttat tgcctgaaga tgagctcgcg    180
   tccgattagc tagttggcgg ggtaaaggcc caccaaggcg gcgatcggta gctggtctga    240
   gaggatgagc agccacactg ggactgagac acggcccaga ctcctacggg aggcagcagt    300
   ggggaatttt ccgcaatggg cgaaagcctg acggagcaag accgcgtggg ggaggaaggc    360
   tcttgggttg taaacccctt ttctcaggga agaataatga cggtacctga ggaatcagcc    420
   tcggctaact ccgtgccagc agccgcggta atacggagga ggcaagcgtt atccggaatt    480
   attgggcgta aagcgtccgc aggtggctag tcaagtcggt ggtcaaagca cagggctcaa    540
   ctctgtaaag gccggcgaaa ctgacaggct agagtgcggt aggggcagag ggaattcccg    600
   gtgtagcggt gaaatgcgta gatatcggga agaacaccag tagcgaaagc gctctgctgg    660
   accgcaactg acactcaggg acgaaagcta ggggagcgaa tgggattaga taccccagta    720
   gtcctagccg taaacgatgg atactaggtg ttgtctgtat cgacccggac agtgccggag    780
   ctaacgcgtt aagtatcccg cctggggagt acgcacgcaa gtgtgaaact caaaggaatt    840
   gacgggggcc cgcacaagcg gtggagtatg tggtttaatt cgatgcaacg cgaagaacct    900
   taccagggct tgacatgtcg cgaaccccgc tgaaaggtgg gggtgccttc gggagcgcga    960
   acacaggtgg tgcatggctg tcgtcagctc gtgtcgtgag atgttgggtt aagtcccgca   1020
   acgagcgcaa ccctcgtcct tagttgccat cattaagttg ggcactctag ggagaccgcc   1080
   ggtgataaac cggaggaagg tggggacgac gtcaagtcag catgcccctt acgccctggg   1140
   ctacacacgt actacaatgg tcaggacaga gggccgccaa ctcgcgagag agagctaatc   1200
   ccgaaaacct ggcctcagtt cagattgaag tttgcaactc agcttcatga aggcggaatc   1260
   gctagtaatc gcaggtcagc atactgcggt gaatgcgttc ccgggccttg tacacaccgc   1320
   ccgtcacacc atggaagttg gccatgcccg aagtcattac tctaacccgt atgggaggag   1380
   ggtgccgaag gcagggctga tgactgggga ggtg 1414